# EUROPÄISCHE PATENTSCHRIFT

(11) **EP 1 233 749 B1**
(45) Veröffentlichungstag und Bekanntmachung des Hinweises auf die Patenterteilung: **04.08.2004**
(21) Anmeldenummer: 00985074.4
(22) Anmeldetag: 21.11.2000
(51) Int. Cl.: A61K 7/50, A61K 7/48, B05B 11/00, B05B 7/00

(54) **HANDWASCHLOTION FÜR WIEDERBEFÜLLBARE SCHAUMERZEUGUNGSVORRICHTUNG**
HAND WASHING LOTION FOR A REFILLABLE FOAM-PRODUCING DEVICE
LOTION POUR LE LAVAGE DES MAINS DESTINEE A UN DISPOSITIF DE PRODUCTION DE MOUSSE RECHARGEABLE

(30) Priorität: 01.12.1999 DE 19957739
(43) Veröffentlichungstag der Anmeldung: 28.08.2002
(73) Patentinhaber: Ecolab Inc., St. Paul, MN 55102-1390 (US)
(72) Erfinder: BIERING, Holger, 41516 Grevenbroich (DE); MEYER, Bernhard, 40822 Mettmann (DE); DENZIN, Silke, 40764 Langenfeld (DE)
(74) Vertreter: Sternagel, Fleischer, Godemeyer & Partner Patentanwälte
(86) Internationale Anmeldenummer: PCT/EP2000/011586
(87) Internationale Veröffentlichungsnummer: WO 2001/039728

(56) Entgegenhaltungen:
- EP-A- 0 640 285
- WO-A-95/09605
- WO-A-96/06152
- WO-A-98/53036
- US-A- 4 920 100
- US-A- 5 336 500
- US-A- 5 968 539

## Beschreibung

Die Erfindung betrifft die Verwendung einer Handwaschlotion zum Nachfüllen einer wiederbefüllbaren Schaumerzeugungsvorrichtung, die zur Erzeugung einer schaumförmigen Handwaschlotion dient.

Handwaschlotionen mit einem Gehalt an Natriumlaurylethersulfat und antimikrobiellen Wirkstoffen sind beispielsweise aus der WO-A-9 853 036 bekannt.

Handwaschlotionen zur Erzeugung von schaumförmigen Handwaschlotionen sind in nur einmal befüllbaren Schaumerzeugungsvorrichtungen im Markt. Bei diesen einmal befüllbaren Vorrichtungen besteht speziell in mikrobiologisch sensiblen Anwendungsbereichen, wie z.B. im Krankenhaus, die Gefahr der Keimübertragung über die berührten Oberflächen. Außerdem hat die Vorrichtung den Nachteil, daß sie unter Berücksichtigung ökologischer Aspekte eine Belastung für die Umwelt darstellt.

Obwohl die Nachteile von nicht wiederbefüllbaren Schaumerzeugungsvorrichtungen zur Erzeugung von schaumförmigen Handwaschlotionen offensichtlich sind, wurden in der Vergangenheit keine Handwaschlotionen zum Nachfüllen von wiederbefüllbaren Schaumerzeugungsvorrichtungen zur Erzeugung von schaumförmigen Handwaschlotionen in mikrobiologisch sensiblen Bereichen verwendet. Der Grund hierfür ist darin zu sehen, daß in wiederbefüllbaren Schaumerzeugungsvorrichtungen vielfältige Kontaminationsrisiken und -herde existieren.

Beispielsweise wird zur Erzeugung von Schaum Luft von außen in die Handwaschlotion eingetragen, so daß grundsätzlich das Risiko besteht, daß mit der Luft Keime in die Handwaschlotion eingeschleppt werden. Zweitens besteht beim Nachfüllen von Handwaschlotionen das Risiko, daß während des Umfüllvorgangs Keime eingeschleppt werden. Drittens ist bei mehrfacher Wiederbefüllung der Schaumerzeugungsvorrichtung im Laufe der Zeit mit Ablagerungen, Verkrustungen und anderen Keimnestem zu rechnen. Dort können sich im ungünstigsten Fall Keime ungestört vermehren und bei der Anwendung der Schaumerzeugungsvorrichtung zu einer kontaminierten schaumförmigen Handwaschlotion führen. Aufgrund dieser und anderer Überlegungen gibt es in mikrobiologisch sensiblen Anwendungbereichen, wie z.B. im Krankenhausbereich bisher keine wiederbefüllbaren Schaumerzeugungsvorrichtungen für Handwaschlotionen. Das ist dadurch zu erklären, daß der Fachmann nicht davon ausgehen konnte, daß dieses Problem mit üblichen Mitteln gelöst werden könnte.

Die Aufgabe der vorliegenden Erfindung war es, eine Handwaschlotion für die Verwendung in einer wiederbefüllbaren Schaumerzeugungsvorrichtung zum Erzeugen einer schaumförmigen Handwaschlotionen zur Verfügung zu stellen, wodurch insbesondere im hygienisch sensiblen Bereich, vorzugsweise im Krankenhaus, das mikrobiologische Risiko auch nach mehrfacher Wiederbefüllung nicht wächst.

Dementsprechend ist Gegenstand der vorliegenden Erfindung die Verwendung einer Handwaschlotion, die
a) Fettalkoholethersulfat und/oder Fettalkoholsulfat, vorzugsweise 2 bis 15 Gew.% bezogen auf die gesamte Handwaschlotion, und
b) antimikrobielle Komponenten
enthält, zum Nachfüllen der wiederbefüllbaren Schaumerzeugungsvorrichtung (1), die zur Erzeugung einer schaumförmigen Handwaschlotion dient. Die erfindungsgemäße Verwendung einer derartigen Handwaschlotion ist in Beispiel 1 dargestellt.

### Beispiel 1

Schaumerzeugungsvorrichtungen, wie vorher beschrieben, werden befüllt mit Mischungen, welche folgende Stoffe enthalten:

| **Inhaltsstoffe** | **Mischung A** | **Mischung B** |
|---|---|---|
| Laurylethersulfat-Na | 7,5 % | 7,5 % |
| Alkyl(C₁₂₋₁₆)-1,4-glucosid | 2,5 % | 2,5 % |
| Ethoxylierter Fettsäuremonodiester mit Glycerin | 1 % | 1 % |
| Fettalkoholethoxylat 2,5 EO (C₁₂-C₁₄) | 0,5 % | 0,5 % |
| Natriumbenzoat | 0,5 % | - |
| Citronensäure | 0,3 % | - |
| **pH-Wert** | **5,0** | **5,7** |

Aus den Schaumerzeugungsvorrichtungen wird 10 mal täglich über einen Zeitraum von 4 Wochen (an 5 Tagen pro Woche) über den Tag verteilt Produkt entnommen. Zu Beginn und Ende des Versuchsabschnitts wird der Keimstatus in beiden Lösungen nach dem Oberflächenkeimzahlverfahren auf Agarplatten bestimmt. Danach werden die Schaumerzeugungsvorrichtungen mit den jeweiligen Mischungen aufgefüllt. Im Anschluß wird der vorher beschriebene Versuchsablauf weitere 3 mal durchgeführt.

Die Ergebnisse sind in nachfolgender Tabelle zusammengefaßt:

| | **Keimstatus (KBE/g)** | | | | |
|---|---|---|---|---|---|
| Mischung | Anfang | Phase 1 nach 4 Wochen | Phase 2 nach 8 Wochen | Phase 3 nach 12 Wochen | Phase 4 nach 16 Wochen |
| B | <10 | 1,5 × 10² | 7,3 × 10² | 1,8 × 10³ | 1,2 x 10⁴ |
| A | <10 | <10 | <10 | <10 | <10 |

Wie in Beispiel 1 belegt wird, ist es möglich, durch erfindungsgemäße Verwendung von Handwaschlotionen ausreichende Sicherheit bei der Applikation von schaumförmigen Handwaschlotionen, auch im sensiblen Bereich, wie z.B. im Krankenhaus, zu gewährleisten. Gemäß Beispiel 1 nimmt die Anzahl der Keime in der erzeugten schaumförmigen Handwaschlotion auch nach mehrfacher Wiederbefüllung der wiederbefüllbaren Schaumerzeugungsvorrichtung nicht zu.
Die erfindungsgemäß zu verwendende Handwaschlotion enthält bevorzugt zusätzlich ein Alkylpolyglykosid, wobei es besonders bevorzugt ist, daß die erfindungsgemäß zu verwendenden Handwaschlotionen ein Alkylpolyglukosid mit 8 bis 14 C-Atomen in der Alkylgruppe und 1 bis 5 Glukoseeinheiten enthalten und es ganz besonders bevorzugt ist, wenn das Alkylpolyglykosid in Mengen von 0,1 bis 5 Gew.% bezogen auf die gesamte Handwaschlotion vorliegt.

Die wiederbefüllbare Schaumerzeugungsvorrichtung ist für den Einsatz in Einarm- oder Einhebelwandspendevorrichtungen geeignet, wobei eine Schaumerzeugungsvorrichtung (1) vorliegt, die eine Luftpumpkammer (2), einer Flüssigkeitspumpkammer (3), einer mit beiden Kammern (2, 3) mittels Ventilen (16; 23, 24) in Strömungsverbindung bringbaren Mischkammer (11) mit Schaumerzeugungseinrichtung (14) und daran angeschlossenem Betätigungskopf (5) mit Schaumführungskanal (12) und rechtwinklig davon abzweigendem Schaumaustrittskanal (30) enthält, wobei gegen die Kraft einer in der Flüssigkeitspumpkammer (3) angeordneten ersten Feder (19) durch Niederdrükken des Betätigungskopfes (5) ein in der Luftpumpkammer (2) geführter Luftpumpkolben (4) und ein in der Flüssigkeitspumpkammer (3) geführter hohler Flüssigkeitspumpkolben (15) bewegbar sind und der Schaumaustrittskanal (30) auslaßseitig in einem um 55° bis 75°, vorzugsweise 65°, gegenüber dem Schaumaustrittskanal (30) auswärts geneigten Abschnitt (31) endet und daß an dem Flüssigkeitspumpkolben (15) eine zweite, zur ersten Feder (19) gleichgerichtet wirkende Feder (18) angreift.

Bei dieser Schaumerzeugungsvorrichtung für den Einsatz in Einarm- oder Einhebel-Wandspendervorrichtungen endet der Schaumaustrittskanal auslaßseitig in einem um 55° bis 75°, vorzugsweise 65°, gegenüber dem Schaumaustrittskanal auswärts geneigten Abschnitt und greift an dem Flüssigkeitspumpkolben eine zweite, zur ersten Feder gleichgerichtet wirkende Feder an.

Mit dieser Schaumerzeugungsvorrichtung wird erreicht, daß eine solche Vorrichtung in Einarm- oder Einhebel-Wandspendervorrichtungen funktionsfähig einsetzbar ist. Durch die zweite Feder wird eine zusätzliche Kraft geschaffen, die den Betätigungsarm oder -hebel der Spendervorrichtung, die auf den Betätigungskopf der Schaumerzeugungsvorrichtung wirkt, nach Betätigung in seine Ausgangslage zurückbewegt. Durch den um 55° bis 75°, insbesondere vorzugsweise 65° geneigten Endabschnitt des Schaumaustrittskanals wird im Zusammenwirken mit der durch die zweite Feder bewirkten Rückwärtsbewegung des Betätigungshebels bewirkt, daß nach Betätigung der Spendervorrichtung kein Schaum aus dem Austrittskanal nachtropft bzw. keine unerwünschten "Schaumnasen" am Ende des Schaumaustrittskanals entstehen bzw. stehen bleiben.

Insgesamt entsteht bei dieser Schaumerzeugungsvorrichtung ein Schaum, der extrem feinblasig, cremig und besonders stabil ist. Ein solcher Schaum fällt selbst bei Applikation auf fettige bzw. ölige Haut nicht so leicht in sich zusammen.

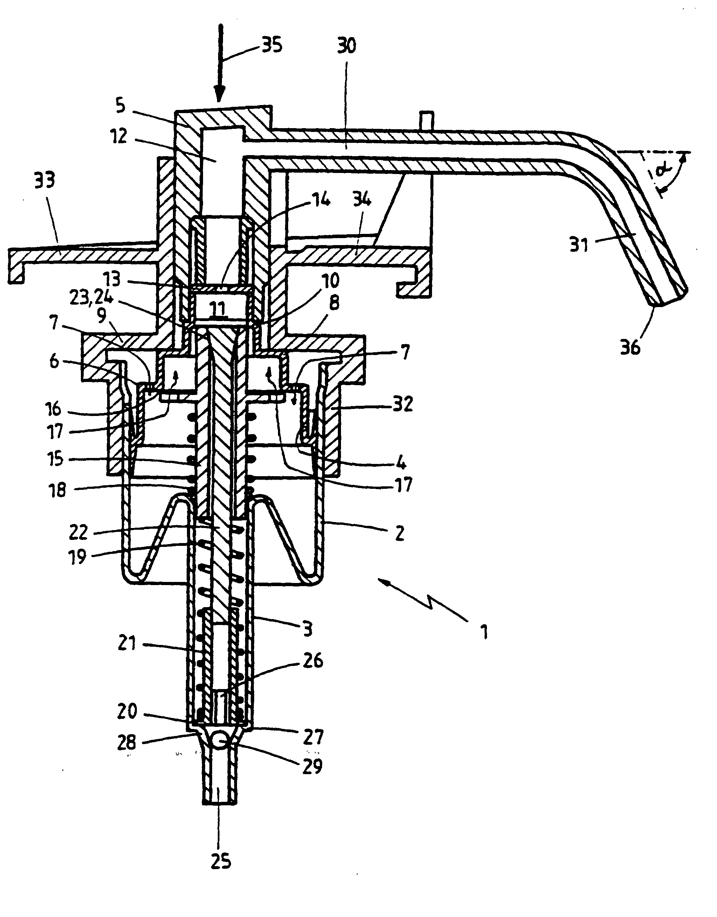

Die nachstehende Zeichnung zeigt in der einzigen Figur eine insgesamt mit 1 bezeichnete Schaumerzeugungsvorrichtung, die im Anwendungsfalle in der Mündung eines nicht dargestellten, flaschenartigen Behältnisses angeordnet ist, welches die mit der Schaumerzeugungsvorrichtung 1 aufzuschäumende Flüssigkeit, vorzugsweise eine Handwaschlotion oder eine antimikrobielle Waschlotion, enthält. Die Schaurnerzeugungsvorrichtung 1 weist eine Luftpumpkammer 2 auf, die einteilig mit einer koaxial zu ihr angeordneten Flüssigkeitspumpkammer 3 ausgebildet ist. Die Luftpumpkammer 2 ist im wesentlichen zylindertopfförmig ausgeformt. In der Luftpumpkammer 2 ist dichtend an der zylindrischen lnnenwand anliegend ein Luftpumpkolben 4 angeordnet. Der Luftpumpkolben 4 ist ebenfalls zylindertopfförmig ausgeformt und weist in Richtung auf den Betätigungskopf 5 zu stufenförmige Durchmesserverringerungen mit entsprechenden zylindrischen Wandabschnitten auf. In einer ersten Stufe 6 des Luftpumpkolbens 4 sind Belüftungslöcher 7 ausgebildet. Luftpumpkolbeninnenseitig ist an diesem Stufenabschnitt ein scheibenförmiges Ventil 16 derart befestigt, daß sich dessen Randbereiche bei entsprechener Druck- bzw. Vakuumbeaufschlagung in Richtung der Pfeile von den anliegenden Wand- und Flächenbereichen abheben können. Mit einer zweiten Stufe 8 liegt der Luftpumpkolben 4 in seiner dargestellten Ausgangslage an einer lnnenwandfläche eines die Luftpumpkammer 2 haltend umfassenden Adapterelementes 9 an. Nach einer dritten Stufe 10 folgt ein zylindrischer Abschnitt des Luftpumpkolbens 4, der eine Mischkammer 11 umfaßt. In Richtung auf einen Schaumführungskanal 12 zu ist die Mischkammer 11 von einem Wandabschnitt 13 mit zentral darin ausgebildeter Schaumerzeugungseinrichtung 14, in diesem Fall eine düsenartige Öffnung, abgeschlossen. Die Schaumerzeugungseinrichtung 14 kann aber auch aus einem Sieb oder zwei mit Abstand zueinander angeordneten Sieben bestehen. An die Schaumerzeugungseinrichtung 14 anschließend ist der Schauführungskanal 12 ausgebildet. Außenseitig umfaßt wird die von dem Luftpumpkolben 4 ausgebildete Mischkammer 11 von einem unteren zylinderförmigen Bereich des Betätigungskopfes 5, der niederdrückbar in einem zylindrischen Bereich des Adapters 9 angeordnet ist. Innenseitig liegt an der dritten Stufe 10 koaxial zu der Schaumerzeugungseinrichtung 14 ausgerichtet ein hohler Flüssigkeitspumpkolben 15 an, der mit seinem entgegengesetzten Ende in die zylinderförmige Flüssigkeitspumpkammer 3 eintaucht. lm Bereich der ersten Stufe 6 des Luftpumpkolbens 4 weist der Flüssigkeitspumpkolben 15 sternförmige Flanschbereiche 17 auf, die mit ihrer äußeren Erstreckung bis in den Bereich der ersten Stufe 6 reichen und die flächig an dem Ringscheibenventil 16 anliegen, wenn sich die Schaumerzeugungsvorrichtung in der in der Figur dargestellten Ausgangsposition befindet. Außenseitig ist der Flüssigkeitspumpkolben 15 unterhalb der Flanschbereiche 17 von einer zweiten Feder 18 umgeben, die mit ihrem den Flanschbereichen 17 abgewandten Ende auf einem oberen Umfangsrand der Flüssigkeitspumpkammer 3 aufliegt, in welche der Flüssigkeitspumpkolben 15 eintaucht. Innerhalb der zylinderförmigen Flüssigkeitspumpkammer 3 ist eine erste Feder 19 angeordnet, die an einem ringförmigen Flanschende 20 eines hohlen, zylinderförmigen Flüssigkeitseintrittselementes 21 und dem in die Flüssigkeitspumpkammer 3 eintauchenden Endes des Flüssigkeitspumpkolbens 15 anliegt. Hierbei ist die erste Feder 19 bezüglich ihrer Länge und Federkraft derart dimensioniert, daß sie den Flüssigkeitspumpkolben 15 und damit auch die daran anliegenden Elemente Luftpumpkolben 4 und Betätigungskopf 5 in die in der Figur dargestellte Ausgangsposition bewegt und in dieser gegen die Schwerkraft hält. Sowohl bei der Feder 18 als auch bei der Feder 19 handelt es sich um eine Druckfeder innerhalb des hohlen Flüssigkeitspumpkolbens 15 ist eine innere Stange 22 angeordnet, die mit konischen Dichtflächen 23, die an entsprechend konischen Dichtflächen 24 des Flüssigkeitspumpkolbens 15 anliegen, den Innenraum des Flüssigkeitspumpkolbens 15 und der Flüssigkeitspumpkammer 3 gegenüber der Mischkammer 11 in der dargestellten Ausgangsposition der Schaumerzeugungsvorrichtung abdichten. Mit ihrem entgegengesetzten Ende taucht die Stange 22 in das hohle Flüssigkeitseintrittselement 21 ein, wobie die erste Feder 19 den weder in den Flüssigkeitspumpkolben 15 noch in das Flüssigkeitseintrittselement 21 eintauchenden Bereich umfaßt. Das der Flüssigkeitseintrittsöffnung 25 zugewandte Ende des zylinderförmigen Flüssigkeitseintrittselementes weist im unteren Randbereich seiner Seitenwandung schlitzförmige Öffnungen 26 auf. Im Übergangsbereich zwischen der Flüssigkeitseintrittsöffnung 25 und dem eine Stufe 27 für einen Anschlag des ringförmigen Flanschendes 20 ausbildenden Endes der Flüssigkeitspumpkammer 3 weist diese einen konischen Bereich 28 auf, der mittels darin angeordneter Kugel 29 als Rückschlagventil ausgebildet ist. An die Flüssigkeitseintrittsöffnung 25 ist ein nicht dargestelltes Steigrohr angeschlossen, das bis auf den Boden des die aufzuschäumende Flüssigkeit enthaltenden Behältnisses reicht.

Der Betätigungskopf 5 weist einen rechtwinklig vom Schaumführungskanal 12 abzweigenden Schaumaustrittskanal 30 auf. Dieser Schaumaustrittskanal 30 endet auslaßseitig in einem Abschnitt 31, der gegenüber dem Schaumaustrittskanal 30 um 65° (Winkel α) auswärts geneigt verläuft.

Das Adapterelement 9 weist einen zylinderförmigen Abschnitt 32 auf, dessen Wand dichtend in die Mündung des nicht dargestellten, die aufzuschäumende Flüssigkeit enthaltenden Behältnisses eingreift und an dieser anliegt. In diesem Bereich kann auch ein Gewinde vorgesehen sein, so daß die dargestellte Vorrichtung und das Behältnis zum Wiederbefüllen des Behältnisses voneinander getrennt werden können. Weiterhin weist die Schaumerzeugungsvorrichtung 1 Haltearme oder Halteelemente 33, 34 auf, mit Hilfe welcher sie an einer Einarm- oder Einhebel-Wandspender-Vorrichtung zu befestigen ist.

Zur Abgabe von Schaum wird der Betätigungskopf 5 in Richtung des Pfeiles 35 niedergedrückt. Durch kraftschlüssige Übertragung an der dritten Stufe 10 wird diese Bewegung auf den Luftpumpkolben 4 und den Flüssigkeitspumpkolben 15, der innenseitig an der dritten Stufe 10 und/oder durch unmittelbare Anlage an dem Ventil 16 an der ersten Stufe 6 anliegt, übertragen. Bei Betätigung des Betätigungskopfes 5 in Richtung des Pfeiles 35 bewegt sich somit der Flüssigkeitspumpkolben 15 in die Flüssigkeitspumpkammer 3 hinein, wobei die konischen Dichtflächen 23 und 24 eine ringförmige Flüssigkeitsdurchtrittsöffnung in die Mischkammer 11 freigeben. Dadurch, daß der Flüssigkeitspumpkolben 15 in die Flüssigkeitspumpkammer 3 eintaucht, und aufgrund der damit auf die in der Flüssigkeitspumpkammer 3 befindliche Flüssigkeit ausgeübten Druckes, wird die Kugel 29 in die gezeigte Verschlußstellung gepreßt und die in der Flüssigkeitspumpkammer 3 enthaltene Flüssigkeit in die Mischkammer 11 gefördert. Gleichzeitig wird aber auch durch die Bewegung des Luftpumpkolbens 4 die in der Luftpumpkammer 2 befindliche Luft komprimiert und aufgrund des erhöhten Druckes das Ventil 16 im Bereich der sternförmigen Flanschbereiche 17 in Richtung der dortigen Pfeile leicht umgebogen, so daß gleichzeitig Luft in die Mischkammer 11 gefördert wird. Zu diesem Zeitpunkt liegt der Flüssigkeitspumpkolben 15 nicht mehr an der Stufe 10 an. Das gleichzeitige Eintreten von flüssigkeit und Luft in die Mischkammer 11 und deren Transport durch die Schaumerzeugungseinrichtung 14 führen dazu, daß sich im Schaumführungskanal 12 Schaum bildet. Dieser wird aufgrund des durch die Flüssigkeit und die Luft bei der entsprechenden Bewegung der Kolben erzeugten Druckes durch den Schaumaustrittskanal 13 und dessen Endabschnitt 31 aus der Schaumerzeugungsvorrichtung herausgeführt.

Wird dann der Betätigungskopf 5 wieder losgelassen, treiben die erste Feder 19 und die zweite Feder 18 den Flüssigkeitspumpkolben 15 und den Luftpumpkolben 4 wieder in die in der Figur dargestellte Ausgangsstellung. Bei diesem Vorgang bildet sich in der Luftpumpkammer 2 ein Vakuum, das bewirkt, daß sich das Ventil 16 im Bereich der Belüftungslöcher 7 von diesen in Richtung der Pfeile weg bewegt und damit eine Belüftung der Luftpumpkammer 2 erfolgt. Gleichzeitig wird durch die Bewegung des Flüssigkeitspumpkolbens 15 in der Flüssigkeitspumpkammer 3 ein Unterdruck erzeugt, aufgrund dessen sich die Kugel 29 aus der Verschlußposition nach oben bewegt und Flüssigkeit durch die Flüssigkeitseintrittsöffnung 25 angesaugt wird, die aufgrund der schlitzförmigen Öffnungen 26 die Möglichkeit hat, die Kugel 29 zu umströmen.

Es ist weiterhin bevorzugt, daß die derart oder anders ausgestaltete Schaumerzeugungsvorrichtung Bestandteil einer Einhebel-Wandspendervorrichtung ist.

Vorzugsweise enthält die erfindungsgemäß zu verwendende Handwaschlotion eine oder mehrere antimikrobielle Komponenten ausgewählt aus den Gruppen der Alkohole, Aldehyde, antimikrobiellen Säuren, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, lod, lodophore, Peroxide, wobei es besonders bevorzugt ist, daß die Handwaschlotion als antimikrobielle Komponenten eine oder mehrere Verbindungen ausgewählt aus Ethanol, n-Propanol, i-Propanol, 1,3-Butandiol, Phenoxyethanol, 1,2-Propylenglykol, Glycerin, Benzoesäure, Undecylensäure, Zitronensäure, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-hamstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraaza-tetradecandiimidamid, quaternären Ammoniumverbindungen, Guanidinen, Amphoteren enthält. Insbesondere ist es vorteilhaft, wenn als antimikrobielle Komponenten 0,1 bis 5 Gew.% einer Komponente ausgewählt aus Benzoesäure, Undecylensäure und/oder Zitronensäure und /oder 2,4,4'-Trichlor-2'hydroxydiphenylether enthalten sind.

Es ist weiterhin bevorzugt, daß die erfindungsgemäß zu verwendende Handwaschlotion zusätzlich ein oder mehrere weitere Tenside ausgewählt aus den Gruppen der anionischen, kationischen, nichtionischen, amphoteren Tenside und der Phosphorsäureester und deren Salzen enthalten, wobei es besonders bevorzugt ist, daß die erfindungsgemäß zu verwendenden Handwaschlotionen als weiteres Tensid ein Aminoxid und/oder einen alkoxylierten Alkyl-Alkohol mit 8 bis 22 C-Atomen in der Alkylkette enthalten, besonders bevorzugt aus der Gruppe der gemischten Ethoxylate/Propoxylate von verzweigten oder unverzweigten Alkyl-Alkoholen mit 8 bis 22 C-Atomen in der Alkylkette und der endgruppenverschlossenen Ethoxylate von verzweigten oder unverzweigten Alkyl-Alkoholen mit 8 bis 22 C-Atomen in der Alkylkette und ganz besonders bevorzugt aus der Gruppe ethoxylierter und propoxylierter Alkyl-Alkohole mit 12 bis 22 C-Atomen im Alkylteil, Butylether eines ethoxylierten Alkyl-Alkohols mit 12 bis 22 C-Atomen im Alkylteil und Methylether eines ethoxylierten Alkyl-Alkohols mit 12 bis 22 C-Atomen im Alkylteil.

In einer weiteren bevorzugten Ausführungsform enthalten die erfindungsgemäß zu verwendenden Formulierungen zusätzlich Lösevermittler, wie beispielsweise Cumolsulfonat, einwertige Alkohole, wie Isopropanol, Butanol, Ethanol sowie mehrwertige Alkohole wie Glykole oder Glycerin sowie deren Ether und Ester, wobei unter dem Begriff Lösevermittler auch Tenside, wie Fettaminethoxylate und andere Stoffe mit lösungsvermittelnden Eigenschaften fallen können.

Es ist weiterhin bevorzugt, daß die erfindungsgemäß zu verwendenden Handwaschlotionen als Lösung, Gel, Emulsion, Paste, Dispersion vorliegen.

Es ist ebenso bevorzugt, daß die erfindungsgemäß zu verwendenden Handwaschlotionen in Betrieben oder Einrichtungen, in denen gesetzliche Vorgaben hinsichtlich der Kontaminationsgefahr durch Mikroorganismen bestehen, bzw. es darauf ankommt, daß keine Keimübertragung stattfindet, zum Einsatz kommen. Besonders bevorzugte Einsatzbereiche sind Krankenhäuser, die lebensmittelherstellende und -abfüllende Industrie, die kosmetische und pharmazeutische Industrie sowie Großküchenbetriebe.

## Patentansprüche

1. Verwendung einer Handwaschlotion, die
a) Fettalkoholethersulfat und/oder Fettalkoholsulfat und
b) eine antimikrobielle Komponente
enthält, zum Nachfüllen einer wiederbefüllbaren Schaumerzeugungsvorrichtung, die zur Erzeugung einer schaumförmigen Handwaschlotion dient, wobei eine Schaumerzeugungsvorrichtung (1) vorliegt die eine Luftpumpkammer (2), einer Flüssigkeitspumpkammer (3), einer mit beiden Kammem (2, 3) mittels Ventilen (16; 23, 24) in Strömungsverbindung bringbaren Mischkammer (11) mit Schaumerzeugungseinrichtung (14) und daran angeschlossenem Betätigungskopf (5) mit Schaumführungskanal (12) und rechtwinklig davon abzweigendem Schaumaustrittskanal (30) enthält, wobei gegen die Kraft einer in der Flüssigkeitspumpkammer (3) angeordneten ersten Feder (19) durch Niederdrücken des Betätigungskopfes (5) ein in der Luftpumpkammer (2) geführter Luftpumpkolben (4) und ein in der Flüssigkeitspumpkammer (3) geführter hohler Flüssigkeitspumpkolben (15) bewegbar sind und der Schaumaustrittskanal (30) auslaßseitig in einem um 55° bis 75° gegenüber dem Schaumaustrittskanal (30) auswärts geneigten Abschnitt (31) endet und daß an dem Flüssigkeitspumpkolben (15) eine zweite, zur ersten Feder (19) gleichgerichtet wirkende Feder (18) angreift.

2. Verwendung nach Anspruch 1, **dadurch gekennzeichnet, daß** die Schaumerzeugungsvorrichtung Bestandteil einer Einhebel-Wandspender-Vorrichtung ist.

3. Verwendung nach Anspruch 1 oder 2, **dadurch gekennzeichnet, daß** die Handwaschlotion eine oder mehrere antimikrobielle Komponenten ausgewählt aus den Gruppen der Alkohole, Aldehyde, antimikrobiellen Säuren, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butylcarbamat, lod, lodophore, Peroxide enthält.

4. Verwendung nach Anspruch 3, **dadurch gekennzeichnet, daß** eine oder mehrere antimikrobielle Komponenten in einer Konzentration von 0,05 bis 20 Gew.% bezogen auf die gesamte Handwaschlotion enthalten sind.

5. Verwendung nach Anspruch 4, **dadurch gekennzeichnet, daß** die Handwaschlotion als antimikrobielle Komponenten eine oder mehrere Verbindungen ausgewählt aus Ethanol, n-Propanol, i-Propanol, 1,3-Butandiol, Phenoxyethanol, 1,2-Propylenglykol, Glycerin, Benzoesäure, Undecylensäure, Zitronensäure, 2-Benzyl-4-chlorphenol, 2,2-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid. N,N'-Bis-4(-Chlorphenyl)-3,12-diimino-2,4,11,13-tetraaza-tetradecandiimidamid, quaternären Ammoniumverbindungen, Guanidinen, Amphoteren enthält.

6. Verwendung nach Anspruch 5, **dadurch gekennzeichnet, daß** die Handwaschlotion als antimikrobielle Komponenten 0,1 bis 5 Gew.% einer Komponente ausgewählt aus Benzoesäure. Undecylensäure und/oder Zitronensäure und /oder 2,4,4'-Trichlor-2'-hydroxydiphenylether enthält.

7. Verwendung nach einem oder mehreren der Ansprüche 1 bis 6, **dadurch gekennzeichnet, daß** die Handwaschlotion 2 bis 15 Gew.-% Fettalkoholethersulfat und/oder Fettalkoholsulfat enthält.

8. Verwendung nach einem oder mehreren der Ansprüche 1 bis 7, **dadurch gekennzeichnet, daß** die Handwaschlotion zusätzlich ein Alkylpolyglycosid enthält.

9. Verwendung nach Anspruch 8, **dadurch gekennzeichnet, daß** die Handwaschlotion 0,1 bis 5 Gew.-% Alkylpolyglycosid enthält.

10. Verwendung nach einem oder mehreren der Ansprüche 1 bis 9, **dadurch gekennzeichnet, daß** die Handwaschlotion weitere Komponenten ausgewählt aus den Gruppen der Tenside und Lösevermittler enthält.

11. Verwendung nach einem oder mehreren der Ansprüche 1 bis 10 in Betrieben oder Einrichtungen, in denen gesetzliche Vorgaben hinsichtlich der Kontaminationsgefahr durch Mikroorganismen bestehen.

12. Verwendung nach Anspruch 11 in Krankenhäusern, in der Lebensmittel herstellenden und abfüllenden Industrie, in der kosmetischen und pharmazeutischen Industrie sowie in Großküchen.

## Claims

1. Use of a hand washing lotion, said lotion containing
a) fatty alcohol ether sulfate and/or fatty alcohol sulfate, and
b) an antimicrobial component
for the refilling of a refillable foam-producing device which serves to produce a foam-type hand washing lotion, said device being a foam-producing device (1) which includes an air pump chamber (2), a liquid pump chamber (3), a mixing chamber (11) which can be flow-connected with the above two chambers (2, 3) by means of valves (16; 23, 24) and has a foam-producing unit (14) and an actuating head (5) connected thereto which has a foam-guiding channel (12) and a foam outlet channel (30) branching therefrom at a right angle, wherein an air pump piston (4) guided in the air pump chamber (2) and a hollow liquid pump piston (15) guided in the liquid pump chamber (3) are movable by pressing down the actuating head (5) against the force of a first spring (19) arranged in the liquid pump chamber (3), and the foam outlet channel (30) at the outlet side thereof terminates in a section (31) inclined outwardly by 55° to 75° with respect to the foam outlet channel (30), and wherein a second spring (18) acting in the same direction as the first spring (19) acts on the liquid pump piston (15).

2. The use according to claim 1, **characterized in that** the foam-producing device is a component of a single-lever wall dispenser.

3. The use according to claim 1 or 2, **characterized in that** the hand washing lotion includes one or more antimicrobial components selected from the groups of alcohols, aldehydes, antimicrobial acids, carboxylic esters, acid amides, phenols, phenol derivatives, diphenyls, diphenylalkanes, urea derivatives, oxygen, nitrogen acetals and formals, benzamidines, isothiazolines, phthalimide derivatives, pyridine derivatives, antimicrobial surface-active compounds, guanidines, antimicrobial amphoteric compounds, quinolines, 1,2-dibromo-2,4-dicyanobutane, iodo-2-propynylbutyl carbamate, iodine, iodophors, peroxides.

4. The use according to claim 3, **characterized in that** one or more antimicrobial components are included at a concentration of from 0.05 to 20 wt.-%, relative to the overall hand washing lotion.

5. The use according to claim 4, **characterized in that** the hand washing lotion includes one or more compounds selected from ethanol, n-propanol, isopropanol, 1,3-butanediol, phenoxyethanol, 1,2-propylene glycol, glycerol, benzoic acid, undecylenic acid, citric acid, 2-benzyl-4-chlorophenol, 2,2'-methylenebis(6-bromo-4-chlorophenol), 2,4,4'-trichloro-2'-hydroxydiphenyl ether, N-(4-chlorophenyl)-N-(3,4-dichlorophenyl)urea, N,N'-(1,10-decane-diyldi-1-pyridinyl-4-ylidene)-bis(1-octaneamine) dihydrochloride, N,N'-bis(4-chlorophenyl)-3,12-diimino-2,4,11,13-tetraazatetradecane diimide amide, quaternary ammonium compounds, guanidines, amphoterics, as antimicrobial components.

6. The use according to claim 5, **characterized in that** the hand washing lotion includes 0.1 to 5 wt.-% of a component selected from benzoic acid, undecylenic acid and/or citric acid and/or 2,4,4'-trichloro-2'-hydroxydiphenyl ether as antimicrobial components.

7. The use according to one or more of claims 1 to 6, **characterized in that** the hand washing lotion includes 2 to 15 wt.-% of fatty alcohol ether sulfate and/or fatty alcohol sulfate.

8. The use according to one or more of claims 1 to 7, **characterized in that** the hand washing lotion additionally includes an alkylpolyglycoside.

9. The use according to claim 8, **characterized in that** the hand washing lotion includes 0.1 to 5 wt.-% alkylpolyglycoside.

10. The use according to one or more of claims 1 to 9, **characterized in that** the hand washing lotion includes further components selected from the groups of surfactants and solubilizers.

11. The use according to one or more of claims 1 to 10 in factories or facilities where legal regulations as to the risk of contamination by microorganisms exist.

12. The use according to claim 11 in hospitals, in food-producing and food-packaging industries, cosmetic and pharmaceutical industries, as well as in large kitchens.

## Revendications

1. Utilisation d'une lotion pour le lavage des mains qui contient
a) du sulfate d'éther d'alcool gras et/ou du sulfate d'alcool gras et
b) des composants antimicrobiens
permettant la recharge d'un dispositif de production de mousse rechargeable servant à produire une solution mousseuse pour le lavage des mains, dans un dispositif de production de mousse (1) qui renferme un réservoir d'air (2), un réservoir de liquide (3), une chambre de mélange (11) entrant en liaison d'écoulement avec les deux réservoirs (2, 3) au moyen de soupapes (16 ; 23, 24), ayant un dispositif de production de mousse (14) et, raccordé à celui-ci, un bouton d'actionnement (5) avec un canal de guidage de la mousse (12) et un canal de sortie de la mousse (30) qui en part en angle droit, un piston à air (4) guidé dans le réservoir d'air (2) et un piston à liquide (15) creux guidé dans le réservoir de liquide (3) pouvant se déplacer contre la force d'un premier ressort (19) disposé dans le réservoir de liquide (3) lorsqu'on appuie sur le bouton d'actionnement (5), et le canal de sortie de la mousse (30) se terminant, côté évacuation, par un tronçon (31) incliné vers l'extérieur de 55° à 75° par rapport au canal de sortie de la mousse (30), et avec un deuxième ressort (18) agissant dans le même sens que le premier ressort (19) agit sur le piston à liquide (15).

2. Utilisation selon la revendication 1,
**caractérisée en ce que**
le dispositif de production de mousse fait partie d'un dispositif de distribution mural à levier unique.

3. Utilisation selon la revendication 1 ou 2,
**caractérisée en ce que**
la lotion pour le lavage des mains contient un ou plusieurs composants antimicrobiens choisis dans les groupes des alcools, aldéhydes, acides antimicrobiens, esters de l'acide carboxylique, amides d'acides, phénols, dérivés de phénols, diphényles, diphénylalcanes, dérivés d'urée, acétals ainsi que formals d'oxygène, d'azote, benzamidines, isothiazolines, dérivés de phtalimide, dérivés de pyridine, composés tensioactifs antimicrobiens, guanidines, composés amphotères antimicrobiens, quinolines, 1,2-dibromo-2,4-dicyanobutane, carbamate d'iodo-2-propynyl-butyle, iode, iodophores, peroxydes.

4. Utilisation selon la revendication 3,
**caractérisée en ce qu'**
un ou plusieurs composants antimicrobiens sont contenus dans une concentration de 0,05 à 20 % en poids par rapport à la lotion pour le lavage des mains totale.

5. Utilisation selon la revendication 4,
**caractérisée en ce que**
la lotion pour le lavage des mains contient, en tant que composants antimicrobiens, un ou plusieurs composés choisis parmi l'éthanol, le n-propanol, l'i-propanol, le butane 1,3-diol, le phénoxyéthanol, le 1,2-propylèneglycol, la glycérine, l'acide benzoïque, l'acide undécylénique, l'acide citrique, le 2-benzyl-4-chlorophénol, le 2,2'-méthylène-bis-(6-bromo-4-chlorophénol), l'éther 2,4,4'-trichloro-2'-hydroxydiphénylique, la N-(4-chlorophényl)-N-(3,4-dichlorophényle)-urée, le dihydrochlorure de N,N'-(1,10-décandiyldi-1-pyridinyl-4-ylène)-bis-(1-octanamine), le N,N'-bis-(4-chlorophényle)-3,12-diimino-2,4,11,13-tétraaza-tétradécandiimidamide, des composés d'ammonium quaternaire, des guanidines, des amphotères.

6. Utilisation selon la revendication 5,
**caractérisée en ce que**
la lotion pour le lavage des mains contient, en tant que composants antimicrobiens, 0,1 à 5 % en poids d'un composant choisi parmi l'acide benzoïque, l'acide undécylénique et/ou l'acide citrique et/ou l'éther 2,4,4'-trichloro-2-hydroxydiphénylique.

7. Utilisation selon l'une quelconque ou plusieurs des revendications 1 à 6,
**caractérisée en ce que**
la lotion pour le lavage des mains contient 2 à 15 % en poids de sulfate d'éther d'alcool gras et/ou de sulfate d'alcool gras.

8. Utilisation selon l'une quelconque ou plusieurs des revendications 1 à 7,
**caractérisée en ce que**
la lotion pour le lavage des mains contient en plus un polyglycoside d'alkyle.

9. Utilisation selon la revendication 8,
**caractérisée en ce que**
la lotion pour le lavage des mains contient 0,1 à 5 % en poids de polyglycoside d'alkyle.

10. Utilisation selon l'une quelconque ou plusieurs des revendications 1 à 9,
**caractérisée en ce que**
la lotion pour le lavage des mains contient d'autres composants choisis dans les groupes des tensioactifs et solubilisants.

11. Utilisation selon l'une quelconque ou plusieurs des revendications 1 à 10, dans les entreprises ou établissements dans lesquels il existe des consignes légales concernant le risque de contamination par des microorganismes.

12. Utilisation selon la revendication 11, dans les hôpitaux, l'industrie fabriquant ou soutirant des denrées alimentaires, l'industrie cosmétique et pharmaceutique ainsi que dans les cuisines industrielles.
